# EUROPEAN PATENT SPECIFICATION

(11) **EP 1 762 171 B1**
(45) Date of publication and mention of the grant of the patent: **11.03.2009**
(21) Application number: 06120422.8
(22) Date of filing: 11.09.2006
(51) Int. Cl.: A61B 1/00, A61B 1/05, A61B 5/107, G01B 11/02

(54) **Device, system and method for determining spacial measurements of anatomical objects for in-vivo pathology detection**
Vorrichtung, System und Verfahren zur Erkennung von räumlichen Messungen von anatomischen Objekten zur Detektion von einer Pathologie in vivo
Appareil, système et procédé destinés a determiner des mesures spatiales des objets anatomiques pour la détection d'une pathologie in vivo

(30) Priority: 09.09.2005 US 715159 P
(43) Date of publication of application: 14.03.2007
(73) Proprietor: Given Imaging Ltd., 20692 Yoqneam (IL)
(72) Inventor: Davidson, Tal, 20692, Yoqneam (IL)
(74) Representative: Harrison Goddard Foote

(56) References cited:
- EP-A2- 0 403 399
- DE-A1- 3 629 435
- JP-A- 59 069 721
- US-A- 5 967 968
- US-A1- 2004 127 785

## Description

### FIELD OF THE INVENTION

The present invention relates to the field of in-vivo operations. More specifically, the present invention relates to devices, systems and methods for in-vivo varices detection and imaging. **BACKGROUND OF THE INVENTION**

Bleeding from sources in the gastrointestinal (GI) tract is common, and remains a major cause of morbidity and mortality. Bleeding varices, for example, esophageal varices, may result from dilated veins in the walls of the GI tract. Bleeding varices may be a life threatening complication of increased blood pressure in veins which may cause veins to balloon outward. The vessel may rupture, causing for example vomiting of blood and bloody stools. Internal bleeding varices may be detected using a wired endoscope; however, undergoing such penetrative treatment may be uncomfortable for the patient.
It is known from US 2004/0127785 to provide a method and apparatus for size analysis in an in-vivo imaging system in which a scale may be overlaid on an image in order to give an estimation of an actual size of imaged anatomical objects.

### SUMMARY OF THE INVENTION

Embodiments of the present invention provide a system and method for providing a scale to an anatomical object imaged by an in-vivo sensing device, including collecting an in-vivo image of the anatomical object, and applying a scale to the anatomical object, where the scale provides spatial measurements of the anatomical object, and wherein the scale is rotated to substantially fit the in-vivo image.

Embodiments of the present invention provide a system and method for approximating spatial measurements of an anatomical object imaged by an in-vivo sensing device, including collecting a set of points having an image of the anatomical object, accepting a subset of the set of points approximating the spatial feature of the anatomical object, processing the subset for providing spatial measurements of the subset, and applying a scale to the anatomical object; wherein the scale is rotated so as substantially to fit the image.

### BRIEF DESCRIPTION OF THE DRAWINGS

The principles and operation of the system, apparatus, and method according to the present invention may be better understood with reference to the drawings, and the following description, it being understood that these drawings are given for illustrative purposes only and are not meant to be limiting, wherein:

Fig. 1 is a schematic illustration of an in-vivo imaging system according to an embodiment of the invention;

Fig. 2 is a schematic illustration of a display system according to an embodiment of the invention; and

Figs. 3 and 4 are flow-charts of methods of in-vivo imaging according to an embodiment of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements throughout the serial views.

### DETAILED DESCRIPTION OF THE INVENTION

In the following detailed description, numerous specific details are set forth in order to provide a thorough understanding of the present invention. However, it will be understood by those skilled in the art that the present invention may be practiced without these specific details. In other instances, well-known methods, procedures, and components have not been described in detail so as not to obscure the present invention.

Some embodiments of the present invention are directed to a typically swallowable in-vivo device, e.g., a typically swallowable in-vivo sensing or imaging device. Devices according to embodiments of the present invention may be similar to embodiments described in United States Patent Application Number 09/800,470, entitled "Device and System for In-vivo Imaging", filed on 8 March, 2001, published on November 1, 2001 as United States Patent Application Publication Number 2001/0035902, and/or in United States Patent Number 5,604,531 to Iddan et al., entitled "In-Vivo Video Camera System", and/or in United States Patent Application Number 10/046,541, filed on January 16, 2002, published on August 15, 2002 as United States Patent Application Publication Number 2002/0109774. An external receiver/recorder unit, a processor and a display, e.g., in a workstation, such as those described in the above publications, may be suitable for use with some embodiments of the present invention. Devices and systems as described herein may have other configurations and/or other sets of components. For example, the present invention may be practiced using an endoscope, needle, stent, catheter, etc. Some in-vivo devices may be capsule shaped, or may have other shapes, for example, a peanut shape or tubular, spherical, conical, or other suitable shapes.

Embodiments of the in-vivo device are typically autonomous and are typically self-contained. For example, the in-vivo device may be or may include a capsule or other unit where all the components are substantially contained within a container, housing or shell, and where the in-vivo device does not require any wires or cables to, for example, receive power or transmit information. The in-vivo device may communicate with an external receiving and display system to provide display of data, control, or other functions. For example, power may be provided by an internal battery or a wireless receiving system. Other embodiments may have other configurations and capabilities. For example, components may be distributed over multiple sites or units. Control information may be received from an external source.

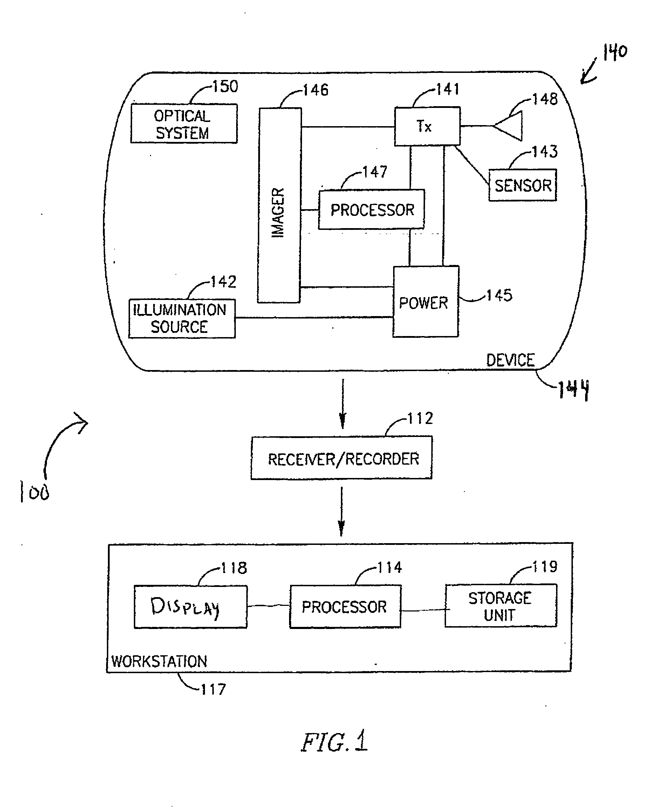

Reference is made to Fig. 1, which is a schematic illustration of an in-vivo imaging system according to an embodiment of the invention. One or more components of an in-vivo system 100 may be used in conjunction with, or may be operatively associated with, the devices and/or components described herein or other in-vivo devices in accordance with embodiments of the invention.

In some embodiments, system 100 may include a device 140 having a sensor, e.g., an imager 146, one or more illumination sources 142, a power source 145, and a transmitter 141. In some embodiments, device 140 may be implemented using a swallowable capsule, but other sorts of devices or suitable implementations may be used. Outside a patient's body may be, for example, an external receiver/recorder 112 (including, or operatively associated with, for example, one or more antennas, or an antenna array), a storage unit 119, a processor 114, and a display 118. In some embodiments, for example, processor 114, storage unit 119 and/or display 118 may be implemented as a workstation 117, e.g., a computer or a computing platform.

Transmitter 141 may operate using radio waves; but in some embodiments, such as those where device 140 is or is included within an endoscope, transmitter 141 may transmit/receive data via, for example, wire, optical fiber and/or other suitable methods. Other known wireless methods of transmission may be used. Transmitter 141 may include, for example, a transmitter module or sub-unit and a receiver module or sub-unit, or an integrated transceiver or transmitter-receiver.

Device 140 typically may be or may include an autonomous swallowable capsule, but device 140 may have other shapes and need not be swallowable or autonomous. Embodiments of device 140 are typically autonomous, and are typically self-contained. For example, device 140 may be a capsule or other unit where all the components are substantially contained within a container or shell, and where device 140 does not require any wires or cables to, for example, receive power or transmit information. In some embodiments, device 140 may be autonomous and non-remote-controllable; in another embodiment, device 140 may be partially or entirely remote-controllable.

In some embodiments, device 140 may communicate with an external receiving system (e.g., receiver/recorder 112) and display system (e.g., workstation 117 or display 118) to provide data, control, or other functions. For example, power may be provided to device 140 using an internal battery, an internal power source, or a wireless system able to receive power. Other embodiments may have other configurations and capabilities. For example, components may be distributed over multiple sites or units, and control information or other information may be received from an external source.

In some embodiments display 118 or workstation 117 may provide detection tools and options for investigation of internal findings, for example, bleeding varices or other pathologies as described in detail below.

Device 140 may include an in-vivo video camera, for example, imager 146, which may capture and transmit images of, for example, the GI tract while device 140 passes through the GI lumen. Other lumens and/or body cavities may be imaged and/or sensed by device 140. In some embodiments, imager 146 may include, for example, a Charge Coupled Device (CCD) camera or imager, a Complementary Metal Oxide Semiconductor (CMOS) camera or imager, a digital camera, a video camera, or other suitable imagers, cameras, or image acquisition components.

Imager 146 in device 140 may be operationally connected to transmitter 141. Transmitter 141 may transmit images to, for example, external transceiver or receiver/recorder 112 (e.g., through one or more antennas), which may send the data to processor 114 and/or to storage unit 119. Transmitter 141 may also include control capability, although control capability may be included in a separate component, e.g., processor 147. Transmitter 141 may include any suitable transmitter able to transmit image data, other sensed data, and/or other data (e.g., control data) to a receiving device. Transmitter 141 may also be capable of receiving signals/commands, for example from an external transceiver. For example, in some embodiments, transmitter 141 may include an ultra low power Radio Frequency (RF) high bandwidth transmitter, possibly provided in Chip Scale Package (CSP).

Transmitter 141 may transmit/receive via antenna 148. Transmitter 141 and/or another unit in device 140, e.g., a controller or processor 147, may include control capability, for example, one or more control modules, processing module, circuitry and/or functionality for controlling device 140, for controlling the operational mode or settings of device 140, and/or for performing control operations or processing operations within device 140. According to some embodiments, transmitter 141 may include a receiver which may receive signals (e.g., from outside the patient's body), for example, through antenna 148 or through a different antenna or receiving element. According to some embodiments, signals or data may be received by a separate receiving device in device 140.

Power source 145 may include one or more batteries or power cells. For example, power source 145 may include silver oxide batteries, lithium batteries, other suitable electrochemical cells having a high energy density, or the like. Other suitable power sources may be used. For example, power source 145 may receive power or energy from an external power source (e.g., an electromagnetic field generator), which may be used to transmit power or energy to in-vivo device 140.

In some embodiments, power source 145 may be internal to device 140, and/or may not require coupling to an external power source, e.g., to receive power. Power source 145 may provide power to one or more components of device 140 continuously, substantially continuously, or in a non-discrete manner or timing, or in a periodic manner, an intermittent manner, or an otherwise non-continuous manner. In some embodiments, power source 145 may provide power to one or more components of device 140, for example, not necessarily upon-demand, or not necessarily upon a triggering event or an external activation or external excitement.

Optionally, in some embodiments, transmitter 141 may include a processing unit or processor or controller, for example, to process signals and/or data generated by imager 146. In another embodiment, the processing unit may be implemented using a separate component within device 140, e.g., controller or processor 147, or may be implemented as an integral part of imager 146, transmitter 141, or another component, or may not be needed. The processing unit may include, for example, a Central Processing Unit (CPU), a Digital Signal Processor (DSP), a microprocessor, a controller, a chip, a microchip, a controller, circuitry, an Integrated Circuit (IC), an Application-Specific Integrated Circuit (ASIC), or any other suitable multi-purpose or specific processor, controller, circuitry or circuit. In some embodiments, for example, the processing unit or controller may be embedded in or integrated with transmitter 141, and may be implemented, for example, using an ASIC.

In some embodiments, imager 146 may acquire in-vivo images continuously, substantially continuously, or in a non-discrete manner, for example, not necessarily upon-demand, or not necessarily upon a triggering event or an external activation or external excitement; or in a periodic manner, an intermittent manner, or an otherwise non-continuous manner.

Transmitter 141 may transmit image data continuously, or substantially continuously, for example, not necessarily upon-demand, or not necessarily upon a triggering event or an external activation or external excitement; or in a periodic manner, an intermittent manner, or an otherwise non-continuous manner.

Device 140 may include one or more illumination sources 142, for example one or more Light Emitting Diodes (LEDs), "white LEDs", or other suitable light sources. Illumination sources 142 may, for example, illuminate a body lumen or cavity being imaged and/or sensed. An optional optical system 150, including, for example, one or more optical elements, such as one or more lenses or composite lens assemblies, one or more suitable optical filters, or any other suitable optical elements, may optionally be included in device 140 and may aid in focusing reflected light onto imager 146, focusing illuminated light, and/or performing other light processing operations.

In some embodiments, illumination source(s) 142 may illuminate continuously, or substantially continuously, for example, not necessarily upon-demand, or not necessarily upon a triggering event or an external activation or external excitement. In some embodiments, for example, illumination source(s) 142 may illuminate a pre-defined number of times per second (e.g., two or four times), substantially continuously, e.g., for a time period of two hours, four hours, eight hours, or the like; or in a periodic manner, an intermittent manner, or an otherwise non-continuous manner.

The components of device 140 may be enclosed within a housing 144, e.g., capsule-shaped, oval, or having other suitable shapes. The housing or shell may be substantially transparent or semi-transparent, and/or may include one or more portions, windows or domes which may be substantially transparent or semi-transparent. For example, one or more illumination source(s) 142 within device 140 may illuminate a body lumen through a transparent or semi-transparent portion, window or dome; and light reflected from the body lumen may enter the device 140, for example, through the same transparent or semi-transparent portion, window or dome, or, optionally, through another transparent or semi-transparent portion, window or dome, and may be received by optical system 150 and/or imager 146. In some embodiments, for example, optical system 150 and/or imager 146 may receive light, reflected from a body lumen, through the same window or dome through which illumination source(s) 142 illuminate the body lumen.

Data processor 114 may analyze the data received via external receiver/recorder 112 from device 140, and may be in communication with storage unit 119, e.g., transferring frame data to and from storage unit 119. Data processor 114 may provide the analyzed data to display 118, where a user (e.g., a physician) may view or otherwise use the data. In some embodiments, data processor 114 may be configured for real time processing and/or for post processing to be performed and/or viewed at a later time. In the case that control capability (e.g., delay, timing, etc) is external to device 140, a suitable external device (such as, for example, data processor 114 or external receiver/recorder 112 having a transmitter or transceiver) may transmit one or more control signals to device 140.

Display 118 may include, for example, one or more screens, monitors, or suitable display units. Display 118, for example, may display one or more images or a stream of images captured and/or transmitted by device 140, e.g., images of the GI tract or of other imaged body lumen or cavity. Additionally or alternatively, display 118 may display, for example, control data, location or position data (e.g., data describing or indicating the location or the relative location of device 140), orientation data, and various other suitable data. In some embodiments, for example, both an image and its position (e.g., relative to the body lumen being imaged) or location may be presented using display 118 and/or may be stored using storage unit 119. Display 118 may include, for example, a grid or scale which may allow a user to measure, in absolute or relative terms, and/or to evaluate specific areas in the displayed image, for example, a varix size, or area. In some embodiments display 118 may include options for marking, delineating or defining areas of interests on images presented on display 118. Other systems and methods of storing and/or displaying collected image data and/or other data may be used.

Typically, device 140 may transmit image information in discrete portions. Each portion may typically correspond to an image or a frame; other suitable transmission methods may be used. For example, in some embodiments, device 140 may capture and/or acquire an image once every half second, and may transmit the image data to external receiver/recorder 112. Other constant and/or variable capture rates and/or transmission rates may be used.

Typically, the image data recorded and transmitted may include digital color image data; in alternate embodiments, other image formats (e.g., black and white image data) may be used. In some embodiments, each frame of image data may include 256 rows, each row may include 256 pixels, and each pixel may include data for color and brightness according to known methods. For example, a Bayer color filter may be applied. Other suitable data formats may be used, and other suitable numbers or types of rows, columns, arrays, pixels, sub-pixels, boxes, super-pixels and/or colors may be used.

Optionally, device 140 may include one or more sensors 143, instead of or in addition to a sensor such as imager 146. Sensor 143 may, for example, sense, detect, determine and/or measure one or more values of properties or characteristics of the surrounding of device 140. For example, sensor 143 may include a pH sensor, a temperature sensor, an electrical conductivity sensor, a pressure sensor, or any other known suitable in-vivo sensor.

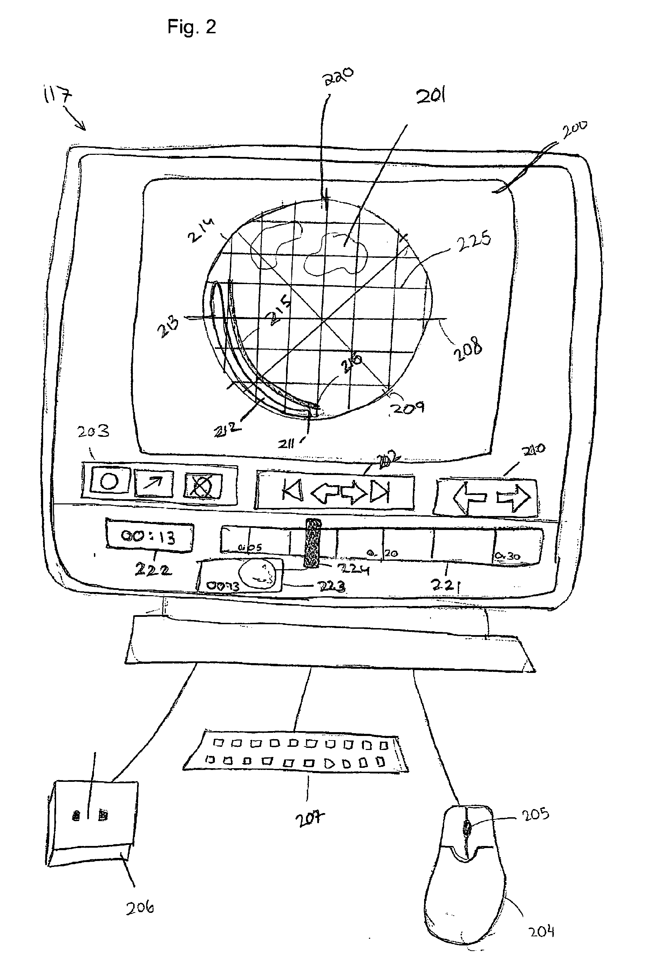

Reference is made to Fig. 2, which is a schematic illustration of a display system according to an embodiment of the invention. Embodiments of the present invention may provide a system and method for providing a scale to an anatomical object imaged by an in-vivo sensing device. An image window 200 may display an image 201. In-vivo sensing device 140 may collect in-vivo image 201 of one or more anatomical objects. Anatomical objects may include, for example, any structure imaged in-vivo by device 140 that is outside device housing 144, for example, structures in the GI tract.

Image 201 may include a still portion or moving portion of a moving image or a captured image of a stream of images. Controls 202 and 203 (preferably in combination with pointing device 204, scrolling wheel 205, keyboard 207 or joystick 206) may alter the display of image 201. Controls 202 may include functionality such as for example play, stop, pause, forward, and backwards for altering a moving image or series of images. Other sets of functionality may be used. In one embodiment, moving the scrolling wheel 205 back and forth allows altering of a moving image display direction.

In some embodiments a clock 222 may display the total time elapse from the beginning of the moving image and a time bar 221 may display the total time elapse from the beginning of the moving image or a period of time, relative to the total elapsed time. A user may be able to create a time stamp 223, relative to time bar 221, which may include the time of a certain captured image and/or a reduced size image showing the image of interests and/or any other suitable annotation with respect to the image. In some embodiments a cursor or an indicator 224 may be move on top of time bar 221. In some embodiments a user may click pointing device 204 (or similarly use joystick 206, keyboard 207 or controls 203 and 210) on a certain point of time bar 221 in which an image of interest appears on image window 200. Any other suitable marking methods may be used.

Workstation 117, including for example a graphics software module, applies a scale to the anatomical object. The scale provides spatial measurements of the anatomical object. Spatial measurements may include any measure or approximate measure of a spatial feature. For example, a measure may include a number of pixels, frames, or bytes, a size of each pixel, any derivation thereof, or any other suitable measure known in the art. Spatial features may include, for example, a shape, size, length, curve, outline, axis, diameter, circumference, angle (e.g., an angle of curvature or rotation), any measure of a coordinate system (e.g., the Cartesian or polar coordinate systems), or any portion or derivation thereof. The scale may include any indicator of spatial measurements, for example, a circumference scale, circular or other grid 225, reference overlay 220, or any other suitable scale that is known in the art.

In one embodiment, device 40 may have a focal length in a predetermined range, for example, from about .5 cm to about 3 cm. Processor 114 may use this known range of focal length to substantially determine the distance between anatomical structures being imaged in image 201 and imager 146. In one embodiment, if the focal length is in the known predetermined range, this distance may be approximated to be constant for all structures being imaged in image 201. In other embodiments, such approximations may not be made. Processor 114 may use the known predetermined to determine spatial measurements throughout image 201 and thus, for all anatomical objects in image 201. The spatial measurements may be provided by a scale, in accordance with embodiments of the invention.

The scale is applied to an imaged anatomical object, for example, on image window 200. In one embodiment, the scale may be displayed adjacent to image 201. In other embodiments, the scale may be displayed peripheral to image 201, preferably on the borders of image 201.

In other embodiments, the scale may be applied to a plurality of anatomical objects, where the scale provides spatial measurements of the anatomical objects, for example, including a relative angle between the anatomical objects and/or a relative size of the anatomical objects.

In one embodiment, image 201 may be displayed, for example, on circular image window 200, and the scale may, for example, be applied along the circumference of the circular image window 200. The circumference scale 220 may include pointers, markers, or grid lines e.g., markers 208, 209, 211, 213, and 214 which may indicate for example the circular angle.

In another embodiment, the scale may include a grid 225, defining a plurality of cells, where the cells provide spatial measurements of the anatomical object. For example, each cell may have a width and a height that are fixed, predetermined or provided measure, for example, a fixed number of pixels. Grid 225 may, for example, be superimposed on image 201.

For example, a grid line may be shown for every 45 degrees, 90 degrees, or any other suitable division of a circle. Other grid lines or overlays may be used, for example, horizontal grid lines, vertical grid lines, or a combination of horizontal, vertical, and circular grid lines may be used.

In other embodiments image 201 may not have round boundaries. Accordingly, a different scale or grid 225 may be applied to the anatomical object. For example horizontal and/or vertical grid lines, or radial lines extending across image 201, or a series of concentric circles, may be used.

The scale is adjusted, for example by a user, or automatically, to substantially fit image 201.

Adjusting the scale includes rotating and translating, or reflecting the scale, or any combination thereof. For example, circumference scale 220 may be rotated from 0 to 360 degrees and positioned, for example, superimposed, at a desired location on image 201. Adjusting and rotating the scale is performed by using a control, for example, control 210, pointing device 204, e.g., a mouse, scrolling wheel 205, keyboard 207, or joystick 206, or a combination thereof. Control 210 may include functionality such as direction in which the scale may be moved or rotated in order to fit image 201. Manipulating control 210 or other controls may be done by using pointing device 204, e.g., a mouse, scrolling wheel 205, keyboard 207, or joystick 206. Other suitable sets of functionality and other suitable controlling devices may be used.

In alternate embodiments, pointing device 204 may control other functions, such as zooming or rotating images and or grid lines. In an exemplary embodiment, when in a certain mode, the user may click and/or hold the wheel 205 of the pointing device 204 (or similarly use joystick 206 or another pointing device) to cause circumference scale 220 or image 201, to rotate. In one embodiment, clicking (e.g., depressing) the scrolling wheel 205 and dragging the pointing device 204 may rotate the circumference scale 220 clockwise or counterclockwise, depending on the dragging direction. In other embodiments, rotation may be achieved in other manners.

While viewing an image, the user may wish to zoom in or out image 201. In one embodiment, rolling the scrolling wheel 205 may zoom in and out image 201, depending on the rolling direction.

In some embodiments a detection of internal findings, for example, internal bleeding, varices, and internal defects may be displayed on image 201 which may be received from, for example, in-vivo device 40 such a swallowable capsule. Specific parameters, for example, size, shape, contour, dimensions and location may be used for diagnostic of a specific finding, for example, internal bleedings, varices and the like. According to some embodiments of the invention, display systems such as those described in reference to Fig. 2 may be used for an evaluation of parameters of image 201. For example, circumference scale 220 or another suitable grid may be moved, resized, or positioned such that for example a line or reference point such as grid line 211 may be positioned on one edge of varix 212 while the other edge may be located at another reference point, such as between grid lines 213 and 214. An evaluation of varix 212 total circumference may be performed by a user, e.g., more than 90 degrees. Evaluation of in-vivo features other than varices may be executed.

A system and method for approximating spatial measurements of an anatomical object imaged by in-vivo sensing device 14, are also provided.

In-vivo sensing device 140 collects a set of points having an image of an anatomical object. Workstation 117 accepts a subset of the set of points, selected by either a user or by software configured for selecting an optimal subset of points for determining the special measurements of the anatomical object being images. The subset of points may include, for example, one or more points contained in the set of points. The subset of points approximates the spatial feature of the anatomical object. For example, the subset of points may follow a length, curve, boundary, axis, line, shape or other spatial features, of the anatomical object either selected by a user or configured by software for this purpose.

For example, a user may use a control 203 in combination with pointing device 204, scrolling wheel 205, keyboard 207, control 210 or joystick 206 to mark areas of interest on image 201. Control 203 may allow the user to select a starting point, for example, point 216 and drawing a line 215, for example, by moving pointing device 204, e.g., a mouse, scrolling wheel 205, keyboard 207 or joystick 206. Line 215 may mark or delineate a certain area of interest e.g., a varix. In some embodiments control 203 may include an "unmark" or cancel or undo button which may cancel the marking line or point (in combination with pointing device 204, scrolling wheel 205, keyboard 207, control 210 or joystick 206). Control 203 may also provide additional information about parameters of line 215, for example, length, circumference, area and the like.

A processor, for example, processor 114, may provide spatial measurements of the subset of points accepted by workstation 117. For example, device 40 may have a focal length in a predetermined range. Processor 114 may use this range of focal length to substantially determine the distance between each point in the subset and imager 146. Processor 114 may use this distance to determine spatial measurements throughout image 201 and thus, the spatial measurements for all points in the subset of points. Processor 114 may use the spatial measurements of each point in the subset to determine spatial measurements of the subset.

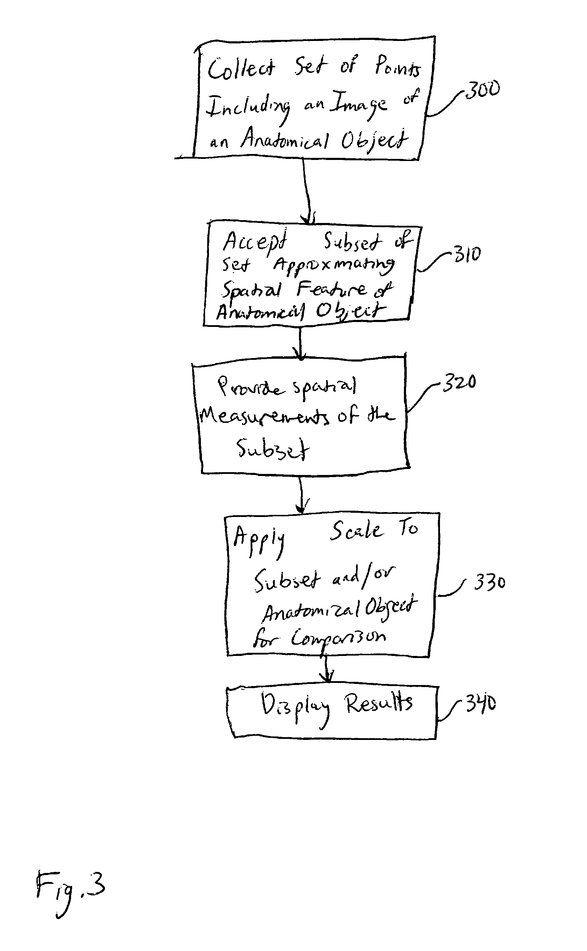

Fig. 3 is a flow-chart of a method of in-vivo detection and imaging in accordance with some embodiments of the invention. The method may be used, for example, in conjunction with one or more components, devices and/or systems described herein, and/or other suitable in-vivo devices and/or systems.

In operation 300, an in-vivo sensing device collects a set of points including an image of an anatomical object. The image may include a still portion or moving portion of a moving image or a captured image of a stream of images. Controls may be used to access, for viewing, a desired image or image stream.

In operation 310, a workstation accepts a subset of the set of points, which may be for example, selected by a user, using controls. The subset of points approximates the spatial feature of the anatomical object. For example, the subset of points may follow a length, curve, boundary, axis, line, shape or other spatial features, of the anatomical object.

In operation 320, a processor provide spatial measurements of the subset, according to embodiments of the invention.

In operation 330, a workstation additionally applies a scale to the subset and/or the anatomical object, where the scale provides spatial measurements of the subset and/or anatomical object, respectively, according to embodiments of the present invention. The processor may compare the spatial measurements of the subset with the spatial measurements of the anatomical object and provide a relative spatial measurement between the subset and the anatomical object.

In operation 340, a workstation may display results including absolute and/or relative special measurements of the subset and/or anatomical object.

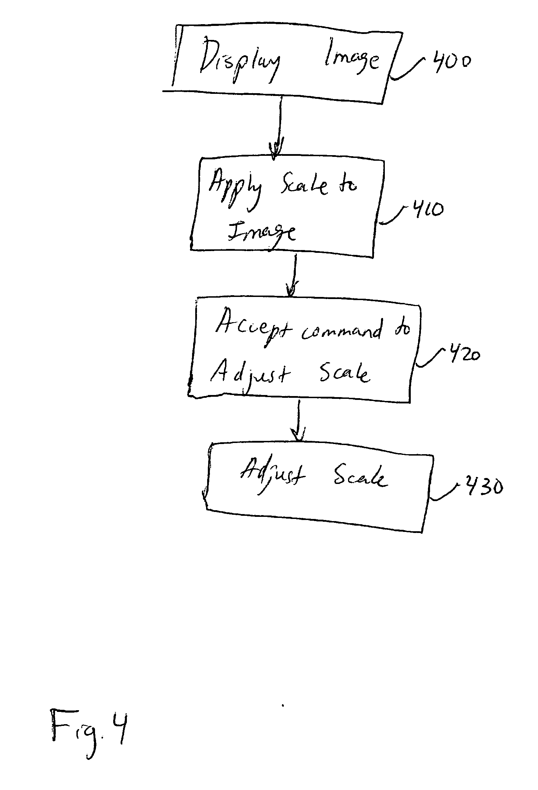

Figure 4 is a flow-chart of a method of in-vivo detection and imaging in accordance with some embodiments of the invention. The method may be used, for example, in conjunction with one or more components, devices and/or systems described herein, and/or other suitable in-vivo devices and/or systems.

In operation 400, an image, image stream, or moving image, including an anatomical object, may be displayed on a display system, for example a monitor. In one embodiment, for example, the image may be collected by an in-vivo device. The image may be an image of interest, for example, an image which may show pathological findings, bleeding vessels, varices or any other medical findings.

In operation 410, a workstation applies a scale to the image, where the scale provides spatial measurements of the anatomical object, according to embodiments of the present invention.

In operation 420, a workstation may accept a command from a control indicating for the workstation to adjust the scale, for example, relative to the image or the display system. A user may provide the command using a pointing device, for example, a mouse, a joystick, a keyboard or the like. In some embodiments scale adjustment may include for example, drawing a straight line, a circle line or inserting a marking point. Any other suitable scale adjustment methods may be used. If the object displayed in operation 400, for example, on a display, is a moving image, a user may pause the display system before providing the scale adjustment command. In alternate embodiments the display system need not be paused.

In operation 430, the workstation may adjust the scale, for example, by rotating, translating and/or reflecting the scale, according to the command accepted in operation 420. In other embodiments, the workstation may alter the type of scale, for example, from a circumference scale to a grid. In yet another embodiment, the workstation may alter the structure of the grid, for example, by adding or removing more grid lines.

Depending on the scale adjustments, the workstation may apply a new, modified or recalculated scale to the anatomical object, according to embodiments of the present invention. For example, the workstation may re-calculate the spatial measurements of the anatomical object. Other suitable operations of sets of operations may be used.

The embodiments of in-vivo image capture devices and methods described above may be used with such a system and method, but other embodiments of in-vivo image capture devices and methods may be used.

It will be appreciated by persons skilled in the art that the present invention is not limited to what has been particularly shown and described hereinabove.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith

## Claims

1. A method for providing a scale to an anatomical object imaged by an in-vivo sensing device (140), comprising:
collecting an in-vivo image (201) of the anatomical object; and
applying a scale (220,225) to the anatomical object, wherein the scale (220,225) provides spatial measurements of the anatomical object;
**characterized in that** the scale (220,225) is rotated so as substantially to fit the in-vivo image (201).

2. The method of claim 1, wherein the spatial measurements include a length.

3. The method of claim 1 or 2, further comprising applying the scale (220,225) to a plurality of anatomical objects, wherein the spatial measurements include a relative size between the anatomical objects.

4. The method of any preceding claim, wherein the scale (220,225) is displayed adjacent to the image (201).

5. The method of any one of claims 1 to 3, wherein the scale (220,225) is displayed peripheral to the image (201).

6. The method of any preceding claim, further comprising displaying the image (201) in a circular display (200) and displaying the scale (220) along the circumference of said circular display (200).

7. The method of any preceding claim, wherein the scale (225) comprises a grid defining a plurality of cells, wherein the cells provide spatial measurements of the anatomical object.

8. The method of claim 7, wherein the grid (225) is superimposed on the image (201).

9. The method of any preceding claim, further comprising translating or reflecting the scale (220,225) to substantially fit the displayed image (201).

10. The method of any preceding claim, further comprising adjusting the image (201) to substantially fit the displayed scale (220,225).

11. A method for approximating spatial measurements of an anatomical object imaged by an in-vivo sensing device (140), comprising:
collecting a set of points comprising an image (201) of the anatomical object;
accepting a subset of said set of points approximating the spatial feature of the anatomical object;
processing said subset for providing spatial measurements of said subset; and
applying a scale (220,225) to the anatomical object;
**characterized in that** the scale (220,225) is rotated so as substantially to fit the image (201).

12. The method of claim 11, further comprising applying a scale (220,225) to the subset, wherein the scale (220,225) provides spatial measurements of the subset.

13. The method of claim 12, further comprising providing a relative spatial measurement between the subset and the anatomical object.

14. The method of claim 11 or any claim depending therefrom, wherein the subset is selected by a user.

15. The method of claim 14, wherein the subset is selected by marking said set of points.

16. The method of claim 11 any claim depending therefrom, wherein the subset intersects the anatomical object.

17. The method of claim 11 any claim depending therefrom, wherein the spatial measurements include a length.

18. A system (100) for providing a scale to an anatomical object, comprising:
an in-vivo sensing device (140) for collecting an in-vivo image (201) of the anatomical object; and
a workstation (117) for applying a scale (220,225) to the anatomical object,
wherein the scale (220,225) provides spatial measurements of the anatomical object;
**characterized in that** the workstation (117) further comprises a control (210) to rotate the scale (220,225) so as substantially to fit the in-vivo image (201).

19. The system of claim 18, wherein the spatial measurements include a length.

20. The system of claim 18 or 19, wherein the workstation (117) applies the scale (220,225) to a plurality of anatomical objects.

21. The system of any one of claims 18 to 20, wherein the scale (225) comprises a grid defining a plurality of cells, wherein the cells provide spatial measurements of the anatomical object.

22. The system of any one of claims 18 to 21, wherein the workstation (117) translates or reflects the scale (220,225) to substantially fit the displayed image (201).

23. A system (100) for approximating spatial measurements of an anatomical object, comprising:
an in-vivo sensing device (140) for collecting a set of points comprising an image of the anatomical object;
a workstation (117) for accepting a subset of said set of points approximating the spatial feature of the anatomical object; and
a processor (114) for providing spatial measurements of said subset;
wherein the workstation (117) applies a scale (220,225) to the anatomical object, wherein the scale (220,225) provides further spatial measurements of the anatomical object;
**characterized in that** the workstation (117) further comprises a control (210) to rotate the scale (220,225) so as substantially to fit the image (201).

24. The system of claim 23, wherein the workstation (117) applies a scale (220,225) to the subset, wherein the scale (220,225) provides spatial measurements of the subset.

25. The system of any one of claims 23 to 24, wherein the processor (114) provides a relative spatial measurement between the subset and the anatomical object.

26. The system of any one of claims 23 to 25, wherein the subset is selected by a user.

## Patentansprüche

1. Verfahren zur Bereitstellung einer Skala an ein durch eine In-vivo-Messvorrichtung (140) aufgenommenes anatomisches Objekt, aufweisend:
Erfassen einer In-vivo-Aufnahme (201) des anatomischen Objektes; und
Anlegen einer Skala (220, 225) an das anatomische Objekt, wobei die Skala (220, 225) räumliche Maße des anatomischen Objektes bereitstellt;
**dadurch gekennzeichnet, dass** die Skala (220, 225) derart rotiert wird,
dass diese im Wesentlichen auf die In-vivo-Aufnahme (201) passt.

2. Verfahren nach Anspruch 1, wobei die räumlichen Maße eine Länge beinhalten.

3. Verfahren nach Anspruch 1 oder 2, das ferner das Anlegen der Skala (220, 225) an eine Vielzahl von anatomischen Objekten aufweist, wobei die räumlichen Maße eine relative Größe zwischen den anatomischen Objekten beinhalten.

4. Verfahren nach einem der vorherigen Ansprüche, wobei die Skala (220, 225) angrenzend an die Aufnahme (201) angezeigt wird.

5. Verfahren nach einem der Ansprüche 1 bis 3, wobei die Skala (220, 225) peripher zu der Aufnahme (201) angezeigt wird.

6. Verfahren nach einem der vorherigen Ansprüche, das ferner das Anzeigen der Aufnahme (201) in einem kreisförmigen Display (200) und das Anzeigen der Skala (220) entlang des Umfangs des kreisförmigen Displays (200) aufweist.

7. Verfahren nach einem der vorherigen Ansprüche, wobei die Skala (225) ein Raster aufweist, das eine Vielzahl von Zellen definiert, wobei die Zellen räumliche Maße des anatomischen Objektes bereitstellen.

8. Verfahren nach Anspruch 7, wobei die Aufnahme (201) mit dem Raster (225) überlagert wird.

9. Verfahren nach einem der vorherigen Ansprüche, das ferner das Übertragen oder Reflektieren der Skala (220, 225) aufweist, um im Wesentlichen auf die angezeigte Aufnahme (201) zu passen.

10. Verfahren nach einem der vorherigen Ansprüche, das ferner das Adjustieren der Aufnahme (201) aufweist, um im Wesentlichen auf die angezeigte Skala (220, 225) zu passen.

11. Verfahren zur Approximierung von räumlichen Maßen eines durch eine In-vivo-Messvorrichtung (140) aufgenommenen anatomischen Objektes, aufweisend:
Erfassen einer Menge von Punkten, die eine Aufnahme (201) des anatomischen Objektes umfasst;
Akzeptieren einer Untermenge der Menge von Punkten, die das räumliche Merkmal des anatomischen Objektes approximiert;
Verarbeiten der Untermenge zur Bereitstellung von räumlichen Maßen der Untermenge; und
Anlegen einer Skala (220, 225) an das anatomische Objekt;
**dadurch gekennzeichnet, dass** die Skala (220, 225) derart rotiert wird, dass diese im Wesentlichen auf die Aufnahme (201) passt.

12. Verfahren nach Anspruch 11, das ferner das Anlegen einer Skala (220, 225) an die Untermenge aufweist, wobei die Skala (220, 225) räumliche Maße der Untermenge bereitstellt.

13. Verfahren nach Anspruch 12, das ferner das Bereitstellen eines relativen räumlichen Maßes zwischen der Untermenge und dem anatomischen Objekt aufweist.

14. Verfahren nach Anspruch 11 oder einem davon abhängigen Anspruch, wobei die Untermenge durch einen Anwender ausgewählt wird.

15. Verfahren nach Anspruch 14, wobei die Untermenge durch Markieren der Menge von Punkten ausgewählt wird.

16. Verfahren nach Anspruch 11 oder einem davon abhängigen Anspruch, wobei die Untermenge das anatomische Objekt schneidet.

17. Verfahren nach Anspruch 11 oder einem davon abhängigen Anspruch, wobei die räumlichen Maße eine Länge beinhalten.

18. System (100) zur Bereitstellung einer Skala an ein anatomisches Objekt, aufweisend:
eine In-vivo-Messvorrichtung (140) zum Erfassen einer In-vivo-Aufnahme (201) des anatomischen Objektes; und
eine Arbeitsstation (117) zum Anlegen einer Skala (220, 225) an das anatomische Objekt,
wobei die Skala (220, 225) räumliche Maße des anatomischen Objektes bereitstellt;
**dadurch gekennzeichnet, dass** die Arbeitsstation (117) ferner eine Steuerung (210) aufweist, um die Skala (220, 225) derart zu rotieren, dass diese im Wesentlichen auf die In-vivo-Aufnahme (201) passt.

19. System nach Anspruch 18, wobei die räumlichen Maße eine Länge beinhalten.

20. System nach Anspruch 18 oder 19, wobei die Arbeitsstation (117) die Skala (220, 225) an eine Vielzahl von anatomischen Objekten anlegt.

21. System nach einem der Ansprüche 18 bis 20, wobei die Skala (225) ein Raster aufweist, das eine Vielzahl von Zellen definiert, wobei die Zellen räumliche Maße des anatomischen Objektes bereitstellen.

22. System nach einem der Ansprüche 18 bis 21, wobei die Arbeitsstation (117) die Skala (220, 225) überträgt oder reflektiert, um im Wesentlichen auf die angezeigte Aufnahme (201) zu passen.

23. System (100) zur Approximierung von räumlichen Maßen eines anatomischen Objektes, aufweisend:
eine In-vivo-Messvorrichtung (140) zur Erfassung einer Menge von Punkten, die eine Aufnahme des anatomischen Objektes umfasst;
eine Arbeitsstation (117) zum Akzeptieren einer Untermenge der Menge von Punkten, die das räumliche Merkmal des anatomischen Objektes approximiert; und
einen Prozessor (114) zur Bereitstellung von räumlichen Maßen der Untergruppe;
wobei die Arbeitsstation (117) eine Skala (220, 225) an das anatomische Objekt anlegt, wobei die Skala (220, 225) weitere räumliche Maße des anatomischen Objektes bereitstellt;
**dadurch gekennzeichnet, dass** die Arbeitsstation (117) ferner eine Steuerung (210) aufweist, um die Skala (220, 225) derart zu rotieren, dass diese im Wesentlichen auf die Aufnahme (201) passt.

24. System nach Anspruch 23, wobei die Arbeitsstation (117) eine Skala (220, 225) an die Untermenge anlegt, wobei die Skala (220, 225) räumliche Maße der Untermenge bereitstellt.

25. System nach einem der Ansprüche 23 bis 24, wobei der Prozessor (114) ein relatives räumliches Maß zwischen der Untermenge und dem anatomischen Objekt bereitstellt.

26. System nach einem der Ansprüche 23 bis 25, wobei die Untermenge durch einen Anwender ausgewählt wird.

## Revendications

1. Procédé pour fournir une échelle à un object anatomique imagé par un dispositif de détection in vivo (140), comprenant les étapes consistant à :
collecter une image in vivo (201) de l'objet anatomique ; et
appliquer une échelle (220, 225) à l'objet anatomique, l'échelle (220, 225) fournissant des mesures spatiales de l'objet anatomique ;
**caractérisé en ce que** l'échelle (220, 225) est tournée de façon à ajuster sensiblement l'image in vivo (201).

2. Procédé selon la revendication 1, dans lequel les mesures spatiales comprennent une longueur.

3. Procédé selon la revendication 1 ou 2, comprenant en outre l'étape consistant à appliquer l'échelle (220, 225) à une pluralité d'objets anatomiques, les mesures spatiales comprenant une taille relative entre les objets anatomiques.

4. Procédé selon l'une quelconque des revendications précédentes, dans lequel l'échelle (220, 225) est affichée de manière adjacente à l'image (201).

5. Procédé selon l'une quelconque des revendications 1 à 3, dans lequel l'échelle (220, 225) est affichée de manière périphérique à l'image (201).

6. Procédé selon l'une quelconque des revendications précédentes, comprenant en outre les étapes consistant à afficher l'image (201) dans un affichage circulaire (200) et afficher l'échelle (220) le long de la circonférence dudit affichage circulaire (200).

7. Procédé selon l'une quelconque des revendications précédentes, dans lequel l'échelle (225) comprend une grille définissant une pluralité de cellules, les cellules fournissant des mesures spatiales de l'objet anatomique.

8. Procédé selon la revendication 7, dans lequel la grille (225) est superposée sur l'image (201).

9. Procédé selon l'une quelconque des revendications précédentes, comprenant en outre l'étape consistant à translater ou réfléchi l'échelle (220, 225) pour ajuster sensiblement l'image affichée (201).

10. Procédé selon l'une quelconque des revendications précédentes, comprenant en outre l'étape consistant à ajuster l'image (201) pour ajuster sensiblement l'échelle affichée (220, 225).

11. Procédé pour donner des valeurs approchées de mesures spatiales d'un objet anatomique imagé par un dispositif de détection in vivo (140), comprenant les étapes consistant à :
collecter un jeu de points comprenant une image (201) de l'objet anatomique ;
accepter un sous-ensemble dudit jeu de points donnant une valeur approchée de la particularité spatiale de l'objet anatomique ;
traiter ledit sous-ensemble pour fournir des mesures spatiales dudit sous-ensemble ; et
appliquer une échelle (220, 225) à l'objet anatomique ;
**caractérisé en ce que** l'échelle (220, 225) est tournée de façon à ajuster sensiblement l'image (201).

12. Procédé selon la revendication 11, comprenant en outre l'étape consistant à appliquer une échelle (220, 225) au sous-ensemble, l'échelle (220, 225) fournissant des mesures spatiales du sous-ensemble.

13. Procédé selon la revendication 12, comprenant en outre l'étape consistant à fournir une mesure spatiale relative entre le sous-ensemble et l'objet anatomique.

14. Procédé selon la revendication 11 ou toute revendication en dépendant, dans lequel le sous-ensemble est sélectionné par un utilisateur.

15. Procédé selon la revendication 14, dans lequel le sous-ensemble est sélectionné en marquant ledit jeu de points.

16. Procédé selon la revendication 11 ou toute revendication en dépendant, dans lequel le sous-ensemble intercepte l'objet anatomique.

17. Procédé selon la revendication 11 ou toute revendication en dépendant, dans lequel les mesures spatiales comprennent une longueur.

18. Système (100) pour fournir une échelle à un objet anatomique, comprenant :
un dispositif de détection in vivo (140) pour collecter une image in vivo (201) de l'objet anatomique ; et
un poste de travail (117) pour appliquer une échelle (220, 225) à l'objet anatomique, l'échelle (220,225) fournissant des mesures spatiales de l'objet anatomique ;
**caractérisé en ce que** le poste de travail (117) comprend en outre une commande (210) pour tourner l'échelle (220, 225) de façon à ajuster sensiblement l'image in vivo (201).

19. Système selon la revendication 18, dans lequel les mesures spatiales comprennent une longueur.

20. Système selon la revendication 18 ou 19, dans lequel le poste de travail (117) applique l'échelle (220,225) à une pluralité d'objets anatomiques.

21. Système selon l'une quelconque des revendications 18 à 20, dans lequel l'échelle (225) comprend une grille définissant une pluralité de cellules, les cellules fournissant des mesures spatiales de l'objet anatomique.

22. Système selon l'une quelconque des revendications 18 à 21, dans lequel le poste de travail (117) translate ou réfléchit l'échelle (220, 225) pour ajuster sensiblement l'image affichée (201).

23. Système (100) pour donner des valeurs approchées de mesures spatiales d'un objet anatomique, comprenant :
un dispositif de détection in vivo (140) pour collecter un jeu de points comprenant une image de l'objet anatomique ;
un poste de travail (117) pour accepter un sous-ensemble dudit jeu de points donnant une valeur approchée de la particularité spatiale de l'objet anatomique ; et
un processeur (114) pour fournir des mesures spatiales dudit sous-ensemble ;
dans lequel le poste de travail (117) applique une échelle (220, 225) à l'objet anatomique, l'échelle (220, 225) fournissant d'autres mesures spatiales de l'objet anatomique ;
**caractérisé en ce que** le poste de travail (117) comprend en outre une commande (210) pour tourner l'échelle (220, 225) de façon à ajuster sensiblement l'image (201).

24. Système selon la revendication 23, dans lequel le poste de travail (117) applique une échelle (220, 225) au sous-ensemble, l'échelle (220, 225) fournissant des mesures spatiales du sous-ensemble.

25. Système selon l'une quelconque des revendications 23 à 24, dans lequel le processeur (114) fournit une mesure spatiale relative entre le sous-ensemble et l'objet anatomique.

26. Système selon l'une quelconque des revendications 23 à 25, dans lequel le sous-ensemble est sélectionné par un utilisateur.
